# EUROPEAN PATENT SPECIFICATION

(11) **EP 2 914 611 B1**
(45) Date of publication and mention of the grant of the patent: **29.08.2018**
(21) Application number: 13785473.3
(22) Date of filing: 01.11.2013
(51) Int. Cl.: C07K 1/30, C12N 15/10, C07K 1/14

(54) **METHOD FOR REMOVAL OF DNA**
VERFAHREN ZUM ENTFERNEN VON DNA
PROCÉDÉ D'ÉLIMINATION D'ADN

(30) Priority: 01.11.2012 EP 12190937
(43) Date of publication of application: 09.09.2015
(73) Proprietor: Novozymes A/S, 2880 Bagsvaerd (DK)
(72) Inventor: HOBEL, Cedric, 2800 Lyngby (DK); KEPKA, Cecilia, Jansson, 218 38 Bunkeflostrand (SE)
(74) Representative: NZ EPO Representatives
(86) International application number: PCT/EP2013/072862
(87) International publication number: WO 2014/068083

(56) References cited:
- WO-A1-00/43502
- WO-A2-2006/073472
- JP-A- 2006 129 861
- US-A1- 2004 219 651

## Description

### FIELD OF THE INVENTION

The present invention relates to a very effective method for obtaining a protein product from a fermentation broth wherein the protein product contains no residual host cell DNA.

### BACKGROUND OF THE INVENTION

Use of aluminium compounds for precipitation of impurities from fermentation broths is known: WO 94/01537 disclosing the use of a soluble aluminate in combination with the simultaneous adjustment of the pH by the addition of an acid; US 3,795,586 discloses a two stage process whereby first non enzymatic components are removed from the fermentation broth by the use of a flocculant, such as an aluminate, and then the soluble enzyme product is precipitated with another flocculant; and WO 96/38469 is disclosing the method of WO 94/01537 in conjunction with additional flocculation agents, clarification and membrane concentration of a solution.

However, it is very difficult to remove residual host cell DNA from a fermentation broth, so there is a desideratum in the art to develop procedures to overcome the problem of residual DNA.

### SUMMARY OF THE INVENTION

It has surprisingly been found that a fermentation broth which has been treated with a poly aluminium chloride contains no, meaning less than 10 ng DNA per gram fermentation broth, i.e. below detection limit, residual host cell DNA, so the present invention claims:
A method for removing residual host cell DNA from a fermentation broth comprising a protein of interest and a microorganism producing the protein of interest, said method comprising:
a) adding a poly aluminium chloride to the fermentation broth, and
b) separating the flocculated microorganism from the fermentation broth.

A method for removing residual host cell DNA from a fermentation broth comprising a protein of interest and a microorganism producing the protein of interest, said method comprising:
a) separating the microorganism from the fermentation broth, and
b) adding a poly aluminium chloride to the fermentation broth.

A method for removing residual host cell DNA from a fermentation broth and subsequent downstream process liquids comprising a protein of interest and a microorganism producing the protein of interest, said method comprising:
a) adding a poly aluminium chloride to the fermentation broth and to the subsequent downstream process liquids,
b) separating the flocculated microorganism from the fermentation broth and
c) refining the subsequent downstream process liquid after adding the poly aluminium chloride.

### DESCRIPTION OF THE DRAWINGS

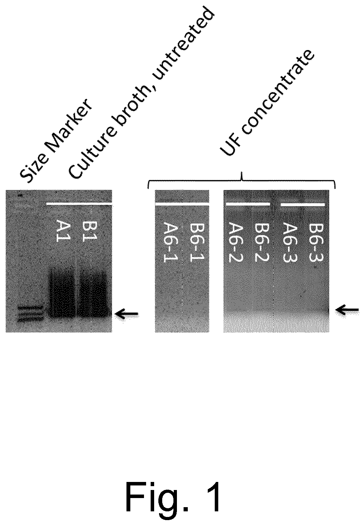

Fig. 1 discloses an agarose gel electrophoresis of PCR-amplified residual DNA fragments from the untreated culture broths and ultrafiltration concentrates (UF concentrate) of GC850™ flocculated samples of Example 1. The presence of residual DNA is indicated by a band on the gel (arrow) in the respective lanes. The intensity of the PCR band is directly linked to the amount of residual DNA present in the sample.

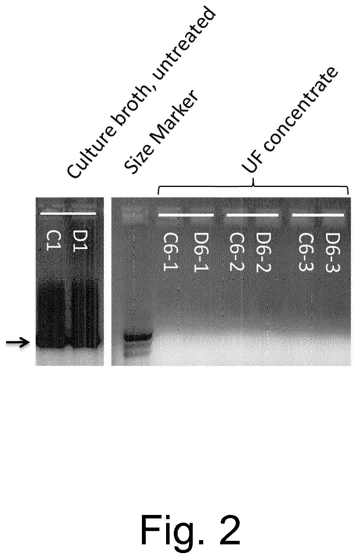

Fig. 2 discloses an agarose gel electrophoresis of PCR-amplified residual DNA fragments from the untreated culture broths and ultrafiltration concentrates (UF concentrate) of NordPAC18™ flocculated samples of Example 1. The presence of residual DNA is indicated by a band on the gel (arrow) in the respective lanes.

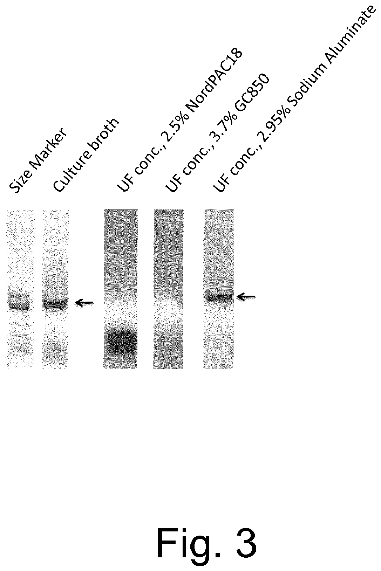

Fig. 3 discloses agarose gel electrophoresis of PCR-amplified residual DNA fragments from the untreated culture broths and ultrafiltration concentrates (UF concentrate) of NordPAC18™, GC850™ and sodium aluminate flocculated samples of Example 2. The presence of residual DNA is indicated by a band on the gel (arrow) in the respective lanes.

### DETAILED DESCRIPTION OF THE INVENTION

The present invention relates to a simple and very effective method for removing residual host cell DNA from a fermentation broth. Within the scope of this invention, host cell DNA is defined as including genomic DNA from the production strain and the fragment of DNA encoding for the protein of interest.

### Microorganism capable of producing the protein of interest

The microbial host cell may be of any genus. The desired protein may be homologous or heterologous to the host cell capable of producing the protein of interest.

The term "homologous protein" means a protein encoded by a gene that is derived from the host cell in which it is produced.

The term "heterologous protein" means a protein encoded by a gene which is foreign to the host cell in which it is produced.

The term "recombinant host cell", as used herein, means a host cell which harbours gene(s) encoding the desired protein and is capable of expressing said gene(s) to produce the desired protein. The desired protein coding gene(s) may be transformed, transfected, transducted, or the like, into the recombinant host cell using techniques well known in the art.

When the desired protein is a heterologous protein, the recombinant host cell capable of producing the desired protein is preferably of fungal or bacterial origin. The choice of recombinant host cell will to a large extent depend upon the gene coding for the desired protein and the source of said protein.

The term "wild-type host cell", as used herein, refers to a host cell that natively harbours gene(s) coding for the desired protein and is capable of expressing said gene(s).

A "mutant thereof" may be a wild-type host cell in which one or more genes have been deleted, e.g., in order to enrich the desired protein preparation.

In a preferred embodiment, the recombinant or wild-type microbial host cell is a bacterium or a fungus.

The microbial host cell may be a yeast cell such as a *Candida, Hansenula, Kluyveromyces, Pichia, Saccharomyces, Schizosaccharomyces,* or *Yarrowia* strain. In another aspect, the strain is a *Saccharomyces carlsbergensis, Saccharomyces cerevisiae, Saccharomyces diastaticus, Saccharomyces douglasii, Saccharomyces kluyveri, Saccharomyces norbensis,* or *Saccharomyces oviformis* strain.

The microbial host cell may be a filamentous fungal strain such as an *Acremonium, Agaricus, Alternaria, Aspergillus, Aureobasidium, Botryospaeria, Ceriporiopsis*, *Chaetomidium, Chrysosporium, Claviceps, Cochliobolus, Coprinopsis, Coptotermes, Corynascus, Cryphonectria, Cryptococcus, Diplodia, Exidia, Filibasidium, Fusarium, Gibberella, Holomastigotoides, Humicola, Irpex, Lentinula, Leptospaeria, Magnaporthe, Melanocarpus, Meripilus, Mucor, Myceliophthora, Neocallimastix, Neurospora, Paecilomyces, Penicillium, Phanerochaete, Piromyces, Poitrasia, Pseudoplectania, Pseudotrichonympha, Rhizomucor, Schizophyllum, Scytalidium, Talaromyces, Thermoascus, Thielavia, Tolypocladium, Trichoderma, Trichophaea, Verticillium, Volvariella,* or *Xylaria* strain.

In another aspect, the strain is an *Acremonium cellulolyticus, Aspergillus aculeatus, Aspergillus awamori, Aspergillus foetidus, Aspergillus fumigatus, Aspergillus japonicus, Aspergillus nidulans, Aspergillus niger, Aspergillus oryzae, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola grisea, Humicola insolens, Humicola lanuginosa, Irpex lacteus, Mucor miehei, Myceliophthora thermophila, Neurospora crassa, Penicillium funiculosum, Penicillium purpurogenum, Phanerochaete chrysosporium, Thielavia achromatica, Thielavia albomyces, Thielavia albopilosa, Thielavia australeinsis, Thielavia fimeti, Thielavia microspora, Thielavia ovispora, Thielavia peruviana, Thielavia setosa, Thielavia spededonium, Thielavia subthermophila, Thielavia terrestris, Trichoderma harzianum, Trichoderma koningii, Trichoderma longibrachiatum, Trichoderma reesei,* or *Trichoderma viride* strain.

In one aspect, the fungal host cell is a strain selected from the group consisting of *Candida, Hansenula, Kluyveromyces, Pichia, Saccharomyces, Schizosaccharomyces, Yarrowia, Acremonium, Aspergillus, Fusarium, Humicola, Mucor, Myceliophthora, Neurospora, Penicillium, Thielavia, Tolypocladium,* and *Trichoderma.*

In a more preferred embodiment, the filamentous fungal host cell is selected from the group consisting of *Trichoderma and Aspergillus* host cells, in particular a strain of *Trichoderma harzianum, Trichoderma koningii, Trichoderma longibrachiatum, Trichoderma reesei, Trichoderma viride*l*, Aspergillus awamori, Aspergillus fumigatus, Aspergillus foetidus, Aspergillus japonicus, Aspergillus nidulans, Aspergillus niger* or *Aspergillus oryzae,* especially a strain of *Trichoderma reesei.*

In another preferred embodiment, the recombinant or wild-type microbial host cell is a bacterium. Examples of microbial host cells include the ones selected from the group comprising gram positive bacteria such as a *Bacillus, Clostridium, Enterococcus, Geobacillus, Lactobacillus, Lactococcus, Oceanobacillus, Staphylococcus, Streptococcus,* or *Streptomyces,* or a Gram-negative bacteria such as a *Campylobacter, Escherichia, Flavobacterium, Fusobacterium, Helicobacter, Ilyobacter*, *Neisseria, Pseudomonas, Salmonella,* or *Ureaplasma.*

In one aspect, the bacterial host cell is a *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* or *Bacillus thuringiensis.*

In another aspect, the bacterial host cell is a *Streptococcus equisimilis, Streptococcus pyogenes, Streptococcus uberis,* or *Streptococcus equi* subspecies *Zooepidemicus.*

In another aspect, the bacterial host cell is a *Streptomyces murinus, Streptomyces achromogenes, Streptomyces avermitilis, Streptomyces coelicolor, Streptomyces griseus,* or *Streptomyces lividans* strain. In another aspect, the bacterial host cell is *Escherichia coli.*

In another aspect, the bacterial host cell is selected from the group consisting of *Bacillus, Streptomyces, Escherichia, Buttiauxella* and *Pseudomonas.*

Strains of these species are readily accessible to the public in a number of culture collections, such as the American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

### Protein of interest

According to the present invention, the protein of interest may be a peptide or a polypeptide. A preferred peptide according to this invention contains from 5 to 100 amino acids; preferably from 10 to 80 amino acids; more preferably from 15 to 60 amino acids. A preferred peptide to be recovered according to the invention is an antimicrobial peptide, a lipopeptide or a another functional peptide like brazzein.

A preferred polypeptide may be any protein that may be produced by a microorganism. The protein may be an enzyme.

In a preferred embodiment, the protein of interest is an enzyme. In a preferred embodiment, the method is applied to a hydrolase (class EC 3 according to Enzyme Nomenclature; Recommendations of the Nomenclature Committee of the International Union of Biochemistry).

In a particularly preferred embodiment, an enzyme selected from the group consisting of an amylase, a protease, a lipase, a cellulase, a xylanase, a mannanase, a phytase, a xylose isomerase, a lactase, an acetolactate decarboxylase, a pectinase, a cutinase, a lyase, an arabinase, a galactanase, an oxidase, a laccase peroxidase and an asparaginase is preferred.

Amylases: An amylase may be the desired enzyme produced according to the invention. Amylases include alpha-amylases, beta-amylases, pullulanases and maltogenic amylases.

An alpha-amylase may be derived from the genus *Bacillus,* such as, derived from a strain of *B. licheniformis, B. amyloliquefaciens, B. subtilis* and *B. stearothermophilus.* Other alpha-amylases include alpha-amylase derived from the strain *Bacillus* sp. NCIB 12289, NCIB 12512, NCIB 12513 or DSM 9375, all of which are described in detail in WO 95/26397, or the alpha-amylase described by Tsukamoto et al., Biochemical and Biophysical Research Communications, 151 (1988), pp. 25-31.

Other alpha-amylases include alpha-amylases derived from a filamentous fungus, preferably a strain of *Aspergillus,* such as, *Aspergillus oryzae* and *Aspergillus niger.*

In a preferred embodiment, the desired enzyme is an alpha-amylase derived from *Aspergillus oryzae* such as the one having the amino acid sequence shown in SEQ ID NO: 10 in WO 96/23874.

The desired enzyme may also be an alpha-amylase derived from *A. niger,* especially the one disclosed as "AMYA_ASPNG" in the Swiss-prot/TeEMBL database under the primary accession no. P56271.

The desired enzyme may also be a beta-amylase, such as any of plants and microorganism beta-amylases disclosed in W.M. Fogarty and C.T. Kelly, Progress in Industrial Microbiology, vol. 15, pp. 112-115, 1979.

The desired enzyme may also be a maltogenic amylase. A "maltogenic amylase" (glucan 1,4-alpha-maltohydrolase, E.C. 3.2.1.133) is able to hydrolyze amylose and amylopectin to maltose in the alpha-configuration. A maltogenic amylase of interest is the one derived from *Bacillus stearothermophilus* strain NCIB 11837. Maltogenic alpha-amylases are described in U.S. Patent Nos. 4,598,048; 4,604,355; and 6,162,628.

Commercially available amylases are Duramyl™, Termamyl SC™, Termamyl Ultra™, Stainzyme™, Natalase™, Novamyl™ and BAN™ (Novozymes A/S), Rapidase™ and Purastar™ (from Genencor International Inc.).

The desired enzyme may also be a pullulanase including glucoamylase (EC 3.2.1.41), which acts on the non-reducing ends of pullulan to produce glucose also termed as a-dextrin 6-glucanohydrolase or true pullulanase or limit dextrinase, pullulanase which acts on a-(1,6)-glucosidic linkage in pullulan to produce maltotriose, isopullulanase, which hydrolyzes a-(1,4)-linkage to produce isopanose, and neopullulanase, which acts on a-(1,4)-linkage to produce panose.

Proteases: Suitable proteases include those of animal, vegetable or microbial origin. Microbial origin is preferred. Chemically modified or protein engineered mutants are included. The protease may be a serine protease or a metallo protease, preferably an alkaline microbial protease or a trypsin-like protease. Examples of alkaline proteases are subtilisins, especially those derived from *Bacillus,* e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the *Fusarium* protease described in WO 89/06270 and WO 94/25583.

Other suitable proteases are the *Nocardiopsis* proteases described in, e.g., WO 2005/115445 useful for pancreatic enzyme replacement.

Suitable commercially available protease enzymes include those sold under the trade names Alcalase®, Duralase™, Durazym™, Relase®, Relase® Ultra, Savinase®, Savinase® Ultra, Primase®, Polarzyme®, Kannase®, Liquanase®, Liquanase® Ultra, Ovozyme®, Coronase®, Coronase® Ultra, Neutrase®, Everlase® and Esperase® (Novozymes A/S), those sold under the tradename Maxatase®, Maxacal®, Maxapem®, Purafect®, Purafect Prime®, Preferenz™, Purafect MA®, Purafect Ox®, Purafect OxP®, Puramax®, Properase®, Effectenz™, FN2®, FN3®, FN4®, Excellase®, Opticlean®, Optimase®, and Excellenz P1000 (Danisco/DuPont), Axapem™ (Gist-Brocases N.V.), BLAP (sequence shown in Figure 29 of US5352604) and variants hereof (Henkel AG) and KAP (*Bacillus alkalophilus* subtilisin) from Kao.

Lipases or cutinases: Suitable lipases and cutinases include those of bacterial or fungal origin. Chemically modified or protein engineered mutant enzymes are included. Examples include lipase from *Thermomyces,* e.g., from *T. lanuginosus* (previously named *Humicola lanuginosa*) as described in EP258068 and EP305216, cutinase from *Humicola,* e.g., *H. insolens* (WO96/13580), lipase from strains of *Pseudomonas* (some of these now renamed to *Burkholderia*), e.g., *P. alcaligenes* or *P. pseudoalcaligenes* (EP218272), *P. cepacia* (EP331376), *P. sp.* strain SD705 (WO95/06720 & WO96/27002), *P. wisconsinensis* (WO96/12012), GDSL-type *Streptomyces* lipases (WO10/065455), Streptomyces griseus (WO11/150157) and *S. pristinaespiralis* (WO12/137147), cutinase from *Magnaporthe grisea* (WO10/107560), cutinase from *Pseudomonas mendocina* (US5,389,536) and lipase from *Thermobifida fusca* (WO11/084412). Other useful lipases may be a *Bacillus* lipase, e.g., from B. *subtilis* (Dartois et al. (1993), Biochemica et Biophysica Acta, 1131, 253-360), B. *stearothermophilus* (JP 64/744992, WO11/084599), *Geobacillus stearothermophilus* lipase (WO11/084417), or *B. pumilus* (WO 91/16422.

Other examples are lipase variants such as those described in EP407225, WO92/05249, WO94/01541, WO94/25578, WO95/14783, WO95/30744, WO95/35381, WO95/22615, WO96/00292, WO97/04079, WO97/07202, WO00/34450, WO00/60063, WO01/92502, WO07/87508 and WO09/109500.

Preferred commercial lipase products include Lipolase™, Lipex™; Lipolex™ and Lipoclean™ (Novozymes A/S), Lumafast (originally from Genencor) and Lipomax (originally from Gist-Brocades).

Still other examples are lipases sometimes referred to as acyltransferases or perhydrolases, e.g., acyltransferases with homology to Candida antarctica lipase A (WO10/111143), acyltransferase from Mycobacterium smegmatis (WO05/56782), perhydrolases from the CE 7 family (WO09/67279), and variants of the M. smegmatis perhydrolase.

Other suitable lipases are the lipases described in, e.g., WO 2006/136159 useful for pancreatic enzyme replacement.

Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme™, Carezyme™, and Celluclean™ (Novozymes A/S), Clazinase™, and Puradax™ HA (DuPont), and KAC-500(B)™ (Kao Corporation).

Xylanases: Xylanases include 1,4-(beta)-D-xylan-xylanohydrolase, (EC 3.2.1.8), 4-xylanohydrolase, endo-1,4-xylanase, endo-1,4-beta-xylanase, beta-1,4-xylanase, endo-1,4-beta-D-xylanase, 1,4-beta-xylan xylanohydrolase, beta-xylanase, beta-1,4-xylan xylanohydrolase, beta-D-xylanase which which degrade the linear polysaccharide beta-1,4-xylan into xylose, thus breaking down hemicellulose, one of the major components of plant cell walls. An example of a a commercially available xylanase is Econase™ (AB Vista).

Asparaginases: Asparaginase also called L-asparaginase and L-asparagine amidohydrolase. (EC 3.5.1.1) is an enzyme that catalyzes the hydrolysis of asparagine to aspartic acid.

Acrylaway™ (Novozyme A/S) is a example of a commercially available asparaginase.

Mannanases: Mannanases (EC 3.2.1.25) include all enzymes catalyzing the hydrolysis of terminal, non-reducing beta-D-mannose in beta-D-mannosides, also called mannanes.

A commercial example of mannanases is Mannaway™ (Novozymes A/S).

Phytases: In the present context a phytase is an enzyme which catalyzes the hydrolysis of phytate (myo-inositol hexakisphosphate) to (1) myo-inositol and/or (2) mono-, di-, tri-, tetra- and/or penta-phosphates thereof and (3) inorganic phosphate.

Phytases include 3-phytase (myo-inositol hexaphosphate 3-phosphohydrolase, EC 3.1.3.8) and 6-phytase (myo-inositol hexaphosphate 6-phosphohydrolase, EC 3.1.3.26).

Examples of commercially available phytases include Ronozyme™ (DSM Nutritional Products), Natuphos™ (BASF), Finase™ (AB Vista), Quantum™ XT and Blue (AB Vista), the Phyzyme™ product series (DuPont) and the Axtra™ PHY product series (DuPont). Other preferred phytases include those described in WO 98/28408, WO 00/43503, and WO 03/066847.

Lyases: The lyase may be a pectate lyase of bacterial or fungal origin. Chemically or genetically modified mutants are included. In a preferred embodiment the pectate lyase is derived from Bacillus, particularly Bacillus substilis, B. licherniformis or B. agaradhaerens, or a variant derived of any of these, e.g. as described in US 6,124,127, WO 1999/027083, WO 1999/027084, WO 2002/006442, WO 2002/092741, WO 2003/095638, Commercially available pectate lyases include XPect; Pectawash™ and Pectaway™ (Novozymes A/S).

Acetolatate decarboxylase (EC 4.1.1.5) belongs to the group of enzymes called lyases, specifically the carboxy-lyases, which cleave carbon-carbon bonds. The commercial enzyme Maturex™ (Novozymes A/S) is used widely in the brewing industry.

Xylose Isomerases: A commercially available xylose isomerase is for example Sweetzyme™ (Novozymes A/S) or GenSweet™ (DuPont).

Lactases: Lactose, a dimer of glucose and galactose is the predominant sugar in milk that is responsible to a large extent to milk intolerance in mammals, especially humans. Beta-galactosidases or Lactases (EC 3.2.1.23) hydrolyse the dimer in the individual carbohydrates thereby increasing the tolerance and ability to digest milk during consumption. Alternative names to lactase include also exo-(1,4)-beta-D-galactanases.
Commercially available lactases include for instance Lactozyme Pure™ (Novozymes A/S) and GODO-YNL2 lactase (DuPont).

Peroxidases/Oxidases: Suitable peroxidases/oxidases include those of plant, bacterial or fungal origin. Chemically modified or protein engineered mutants are included. Examples of useful peroxidases include peroxidases from *Coprinus,* e.g., from C. *cinereus,* and variants thereof as those described in WO 93/24618, WO 95/10602, and WO 98/15257.

Other peroxidases include Guardzyme™ (Novozymes A/S).

### Fermentation broth

The present invention may be useful for any fermentation in industrial scale, e.g., for any fermentation having culture media of at least 50 liters, preferably at least 500 liters, more preferably at least 5,000 liters, even more preferably at least 50,000 liters.

The microorganism producing the protein of interest may be fermented by any method known in the art. The fermentation medium may be a minimal medium as described in, e.g., WO 98/37179, or the fermentation medium may be a complex medium comprising complex nitrogen and carbon sources, wherein the complex nitrogen source may be partially hydrolyzed as described in WO 2004/003216.

The fermentation may be performed as a batch, a repeated batch, a fed-batch, a repeated fed-batch or a continuous fermentation process.

In a fed-batch process, either none or part of the compounds comprising one or more of the structural and/or catalytic elements is added to the medium before the start of the fermentation and either all or the remaining part, respectively, of the compounds comprising one or more of the structural and/or catalytic elements are fed during the fermentation process. The compounds which are selected for feeding can be fed together or separately to the fermentation process.

In a repeated fed-batch or a continuous fermentation process, the complete start medium is additionally fed during fermentation. The start medium can be fed together with or separately from the structural element feed(s). In a repeated fed-batch process, part of the fermentation broth comprising the biomass is removed at regular time intervals, whereas in a continuous process, the removal of part of the fermentation broth occurs continuously. The fermentation process is thereby replenished with a portion of fresh medium corresponding to the amount of withdrawn fermentation broth.

In a preferred embodiment of the invention, a fermentation broth from a fed-batch fermentation process is preferred.

### Flocculation

The method of the invention may be applied to an untreated fermentation broth or to a fermentation broth that has first been subjected to, but not limited to, e.g., a pH adjustment and/or a temperature adjustment. The pH may be adjusted to a pH within a range of pH 2 to pH 11. The pH may be adjusted with any acid or base as known in the art.

According to the present invention, the fermentation broth may be diluted up to 2000% (w/w) with water; preferably the fermentation broth may be diluted 10-2000% (w/w) with water; more preferably the fermentation broth may be diluted 100-1500% (w/w) with water; more preferably the fermentation broth may be diluted 100-1000% (w/w) with water; more preferably the fermentation broth may be diluted 200-800% (w/w) with water.

Dilution with water means, according to the present invention, that the dilution medium may be water, or it may be an ultra filtration permeate from the production of the protein of interest , or it may be a recycle of water or process liquid from the production of the protein of interest, or it may be a condensate from a heater or it may be any combination of the above mentioned, e.g., a mixture of water and an ultra filtration permeate.

A monovalent or divalent salt may then be added to the fermentation broth, in particular a sodium and/or calcium salt and/or a magnesium salt, e.g., sodium chloride, calcium chloride or magnesium chloride. A preferred embodiment is a calcium salt, in particular calcium chloride.

The monovalent or divalent salt may be added to a concentration in the fermentation broth of 0.01-10% (w/w) per kg fermentation broth (un-diluted); preferably 0.5-10% (w/w) per kg fermentation broth (un-diluted); more preferably 1-9% (w/w) per kg fermentation broth (un-diluted); in particular 2-8% (w/w) per kg fermentation broth (un-diluted).

### Aluminium compounds

We have found that poly aluminium chlorides are extremely efficient in removing residual host cell DNA.

Particular useful poly aluminium chlorides include compounds of the formula Alₙ(OH)ₘCl(₃ₙ₋ₘ) and poly aluminium chlorides and aluminium chlorohydrates with the CAS No.: 1327-41-9.

Examples of useful poly aluminium chlorides comprise aluminum chlorohydrate, GC850™ (Al₂(OH)₅Cl) obtainable from Gulbrandsen or NordPac 18 (available from Nordisk Aluminat A/S, Denmark) which is an aluminium complex with the brutto formula Al(OH)_{1,2}Cl_{1,8}. Another example of a useful poly aluminium chloride with the formula Al(OH)_{1,2}Cl_{1,8} is PAX-XL 100 (available from Kemira). Another two examples of useful poly aluminium chloride are PAC (available from Shanghai Haotian Water Treatment Equipment Co., Ltd supplied in solid form) or PAC (available from Tianjin Kairuite technology Ltd. Supplied in liquid form). Another example of useful poly aluminium chloride with the formula Al(OH)_{1,2}Cl_{1,8} is PAX18 (available from Kemira Water Solutions).

The concentration of the poly aluminium chloride will normally be in the range of 0.1-10 % (w/w) calculated per kg fermentation broth (un-diluted); preferably in the range of 0.5-5 % (w/w) calculated per kg fermentation broth (un-diluted).

After addition of the poly aluminium chloride, the pH may be adjusted. The pH may be adjusted to a pH within a range of pH 2 to pH 11. The pH may be adjusted with any acid or base as known in the art.

We claim a method for removing residual host cell DNA from a fermentation broth comprising a protein of interest and a microorganism producing the protein of interest, said method comprising:
a) adding a poly aluminium chloride to the fermentation broth, and
b) separating the flocculated microorganism from the fermentation broth.

The poly aluminium chloride may also be added after the microorganism has been separated from the fermentation broth; so we also claim:
A method for removing residual host cell DNA from a fermentation broth comprising a protein of interest and a microorganism producing the protein of interest, said method comprising:
a) separating the microorganism from the fermentation broth, and
b) adding a poly aluminium chloride to the fermentation broth.

The poly aluminium chloride may also be added in two or more steps: e.g., before the microorganism is removed from the fermentation broth; and then again after the microorganism has been removed such as in the subsequent downstream process liquid.

Thus the invention further comprise a method for removing residual host cell DNA from a fermentation broth and subsequent downstream process liquids comprising a protein of interest and a microorganism producing the protein of interest, said method comprising:
a) adding a poly aluminium chloride to the fermentation broth and to the subsequent downstream process liquids, and
b) separating the flocculated microorganism from the fermentation broth
c) refining the subsequent downstream process liquid after adding the poly aluminium chloride.

### Polymers

Polymers are widely used for particle aggregation. Anionic and cationic polymers are preferred. A useful cationic polymer may be a polyamine, and a useful anionic polymer may be a polyacrylamid. Useful polymer concentrations will normally be in the range of 0.5-20 % (w/w) calculated per kg fermentation broth (un-diluted); preferably in the range of 1-10 % (w/w) calculated per kg fermentation broth (un-diluted).

An example of a useful anionic polymer is Superfloc™ A 130 (Kemira). Examples of useful cationic polymers are Polycat™ (Kemira), C521 (Kemira), and C591 (Kemira).

### Subsequent downstream operations

The flocculated cells may be removed by methods known in the art such as, but not limited to, filtration, e.g., drum filtration, membrane filtration, filter-press dead end filtration, cross-flow filtration, or centrifugation.

The resulting protein solution may then be further processed or refined by methods known in the art. For example, the protein may be recovered by conventional procedures including, but not limited to, further filtration such as ultra-filtration and dia-filtration, extraction, spray-drying, evaporation, precipitation or crystallization. The isolated protein may then be further purified and/or modified by a variety of procedures known in the art including, but not limited to, chromatography e.g. ion exchange, affinity, hydrophobic, chromatofocusing, and size exclusion and/or electrophoretic procedures e.g. preparative isoelectric focusing and/or differential solubility e.g., ammonium sulfate precipitation and/or extraction.

### Detection of residual host cell DNA

The term residual host cell DNA is including genomic DNA from the production strain and the fragment of DNA encoding for the protein of interest.

The detection and quantification of minute amounts of residual host cell DNA may be accomplished by various methods known in the art. Many methods are developed to measure specific single target sequences. Examples of methods are:
- A hybridization-based method for the detection of the specific DNA of defined origin with dot blots and hybridization of radioisotope-labeled DNA probes using random hexamers to generate representative probes covering the whole genome of the host cells.
- A quantitative PCR-based method for the detection of specific DNA of defined origin targeting a specific gene sequence for amplification and calibration using purified, species-matched, genomic DNA.
- A qualitative PCR method for the detection of specific DNA of defined origin targeting a specific gene sequence for amplification and calibration using purified, species-matched, genomic DNA. A threshold is determined based on the lowest amount of genome DNA that can be detected using the method.

According to the present invention purification of trace DNA was accomplished by use of the FastDNA™ Spin Kit (MP Biomedicals). The eluted sample was then subjected to a PCR reaction using specific primers for a chromosomal locus on the host cell. Positive controls are included where known amounts of host DNA is added to PCR reactions in different concentrations. After the PCR reaction the samples are subjected to gel-electrophoresis and intensity of the DNA bands compared to estimate the concentration of the host DNA in the original sample. Detailed protocols for PCR are provided in Innis et aL (1990) PCR Protocols, A Guide to methods and applications, Academic Press inc., N.Y.

By using the method according to this invention there is no detectable residual host cell DNA (less than 10 ng DNA per gram fermentation broth i.e. below detection limit).

The invention is further illustrated in the following examples.

### EXAMPLES

### Example 1

### DNA removal from a fermentation broth

The fermentation broth contained the protein of interest (a maltogenic amylase) and the microorganism producing the amylase of interest (*Bacillus subtilis*). The fermentation was a submerged fermentation as known in the art and the resulting broth had a demonstrated DNA content.

**Control:** Frozen culture broth of the amylase batch was thawed in a water bath. Two independent aliquots of the material were spun down at 10,000 rpm for 20 min., and the resulting supernatants were kept for analysis and will be described here as the "culture broth, untreated" controls.

**Tests:** The fermentation broth was flocculated with four different setups:
Using either GC850™ (Al₂(OH₅)Cl) or NordPAC18™ Al(OH)_{1,2}Cl_{1,8} as the poly aluminate chloride salt, and either Polycat™ or C521™ as the cationic polymer.

The amounts of dilution water, CaCI2, and A130 ™ were kept constant.

The four flocculation setups were performed using combinations of :

| Component | Amount | Stock solution | Concentration * |
|---|---|---|---|
| Culture broth | 500g | - | |
| Tap water | 1500g | - | 300% |
| CaCl2 | 25g | 34% | 5% |
| GC850™ | 10g | 100% | 2% |
| NordPAC18™ | 5g | 100% | 1% |
| C521™ | 40g | 20% | 8% |
| Polycat™ | 50g | 10% | 10% |
| A130™ | 20g | 0.01% | 4% |

| | | | |
|---|---|---|---|
| * These values are calculated respectively to the mass of culture broth to be flocculated. | | | |

The four flocculations described here as A, B, C and D were made using:
**A:** Water, CaCl2, GC850™, C521™, A130™.
**B:** Water, CaCl2, GC850™, Polycat™, A130™.
**C:** Water, CaCl2, NordPAC18™, C521™, A130™.
**D:** Water, CaCl2, NordPAC18™, Polycat™, A130™.

pH was adjusted with acetic acid and/or sodium hydroxide solutions after the addition of the poly aluminate in order to reach the desired pH5.5 set point.

The flocculation solutions were mixed using a magnetic stirrer at room temperature until the desired quality of the flocs were obtained (visual observation). The quality of the respective flocculations was also determined using a turbidity measurement of the supernatant of spun down material (3500 rpm, 5 min). Our experimental data show that comparable turbidity values were obtained for all four solutions.

The flocculated materials were then submitted to a standard lab scale recovery process that includes:
- Centrifugation (3500 rpm, 10 min),
- Polish filtration on a Nutsch apparatus (with HS9000 filter plates, 7.5-18 µm cutoff),
- Germ filtration (with disposable 0.22 µm vacuum filters),
- Ultrafiltration (with a Sartorius filter cassette with 10 kDa cutoff).

Independent triplicate samples were taken at all streams mentioned above and submitted to residual DNA analysis (FastDNA™ Spin Kit and PCR); only the untreated culture broth samples were analyzed as singleton. Single samples taken at all streams were sent for enzyme activity analysis.

### Results

The first two untreated culture broth samples contained a high amount of residual DNA (see arrow on Figure 1). Two of the triplicates of the A and the B samples (A6-2 and A6-3, B6-2 and B6-3), corresponding to the ultrafiltration (UF) concentrated material of the respective A and B flocculations with GC850™, had a positive DNA signal indicated by the presence of a PCR band on an agarose gel (see arrows on Figure 1).
Expectedly, the two other untreated culture broth samples also contained a high amount of residual DNA (see arrow on Figure 2). All triplicates of the C and the D samples (C6-1, C6-2 and C6-3; D6-1, D6-2 and D6-3), corresponding to the ultrafiltration (UF) concentrated material of the respective C and D flocculations with NordPAC18™ had a negative DNA signal indicated by the absence of a PCR band on an agarose gel (no arrows, see Figure 2).

There was no obvious difference between Polycat™ and C521 ™ as cationic polymer in the effect of DNA removal.

Both NordPAC18 and GC850 showed very good ability to remove DNA from a fermentation broth. It was not possible to detect any DNA when using NordPAC18™ and only a small trace of DNA was seen when using GC850™. Activity data support that the exchange of GC850™ for NordPAC18™ has no impact on the concentration of the enzyme in the concentrate.

### Example 2

### DNA removal from a fermentation broth using poly aluminium chlorides compared to sodium aluminate:

The fermentation broth contained an amylase and the microorganism producing the amylase a *Bacillus subtilis.* The fermentation was a submerged fermentation as known in the art and the resulting broth had a demonstrated DNA content.
**Control:** A culture broth of the amylase batch was thawed in a water bath. Three independent aliquots of the material were spun down at 10,000 rpm for 20 min., and the resulting supernatants were kept for analysis and will be described here as the "culture broth, untreated" controls.
**Tests:** GC850™ (Al₂(OH₅)Cl) and NordPAC18™ Al(OH)_{1,2}Cl_{1,8} was tested against a reference sodium aluminate (NaAlO₂, The amounts of dilution water, CaCl₂ were kept constant. The amount of the cationic polymer Polycat™ (Kemira) and anionic polymer A130™ (Kemira) were adapted in order to obtain similar flocculation efficiencies between the samples.

The four flocculation setups were performed using combinations of :

| Component | Amount | Stock solution | Concentration * |
|---|---|---|---|
| Culture broth | 500g | - | |
| Tap water (35°C) | 1500g | - | 300% |
| CaCl2 | 25g | 34% | 5% |
| GC850™ | 18.5g | 100% | 3.7% |
| NordPAC18™ | 12.5g | 100% | 2.5% |
| Sodium aluminate | 14.6g | 30% | 2.95% |
| Polycat™ | 50g | 10% | 10% |
| A130™ | 20g | 0.01% | 4% |

| | | | |
|---|---|---|---|
| * These values are calculated respectively to the mass of culture broth to be flocculated. | | | |

The three flocculations described here as A, B, and C were made using:
**A:** Water, CaCI2, NordPAC18™, Polycat™, A130™.
**B:** Water, CaCI2, GC850™, Polycat™, A130™.
**C:** Water, CaCI2, Sodium aluminate, A130™.

pH was adjusted with acetic acid and/or sodium hydroxide solutions after the addition of the poly aluminate in order to reach the desired pH 5.5 set point.
The flocculation solutions were mixed using a magnetic stirrer until the desired quality of the flocs were obtained (visual observation). The quality of the respective flocculations was also determined using a turbidity measurement of the supernatant of spun down material (3500 rpm, 5 min). The data showed that comparable turbidity values were obtained for all three solutions. The flocculated materials were then submitted to a standard lab scale recovery process that includes:
- Centrifugation (3500 rpm, 10 min),
- Polish filtration on a Nutsch apparatus (with HS9000 filter plates, 7.5-18 µm cutoff),
- Germ filtration (with disposable 0.22 µm vacuum filters),
- Ultrafiltration (with a Sartorius filter cassette with 10 kDa cutoff).

Samples were taken at all streams mentioned above and submitted to residual DNA analysis (FastDNA™ Spin Kit and PCR). Internal controls were performed to assess the quality of PCR results. Single samples taken at all streams were sent for enzyme activity analysis.

### Results:

The three different aluminium salts used under the conditions of the experiment resulted in very similar flocculation quality, as determined by the turbidity of the respective supernatants after total reaction time and centrifugation. The presence of residual DNA was assessed by PCR (see figure 3). The control sample (culture broth) contained substantial amount of DNA (arrow). The UF concentrate of both NordPAC18™ and GC850™ flocculations had negative PCR signal thus it was not possible to detect any DNA (no arrow). The UF concentrate of the sodium aluminate flocculation was positive for residual DNA (arrow).

The results demonstrate that the poly alluminium chlorides NordPAC18™ and GC850™ have a much better DNA removal efficiency than sodium aluminate.

## Claims

1. A method for removing residual host cell DNA from a fermentation broth comprising a protein of interest and a microorganism producing the protein of interest, said method comprising:
a) adding a poly aluminium chloride to the fermentation broth, and
b) separating the flocculated microorganism from the fermentation broth.

2. A method for removing residual host cell DNA from a fermentation broth comprising a protein of interest and a microorganism producing the protein of interest, said method comprising:
a) separating the microorganism from the fermentation broth; and
b) adding a poly aluminium chloride to the fermentation broth.

3. A method for removing residual host cell DNA from a fermentation broth and subsequent downstream process liquids comprising a protein of interest and a microorganism producing the protein of interest, said method comprising:
a) adding a poly aluminium chloride to the fermentation broth and to the subsequent downstream process liquids;
b) separating the flocculated microorganism from the fermentation broth; and
c) refining the subsequent downstream process liquid after adding the poly aluminium chloride.

4. The method according to any of the preceding claims, wherein the protein is an enzyme.

5. The method according to any of the preceding claims, wherein the microorganism is a bacterium or a fungus.

6. The method according to any of the preceding claims, wherein the enzyme is a hydrolase.

7. The method according to any of the preceding claims, wherein the fermentation broth may be diluted up to 2000% (w/w) with water.

8. The method according to any of the preceding claims, wherein the poly aluminium chloride is added at a concentration of 0.1-10 % (w/w) calculated per kg fermentation broth.

9. The method according to any of the preceding claims, wherein one or more flocculating agents are added in addition to the poly aluminium chloride.

10. The method according to claim 9, wherein the flocculating agents are selected from the group consisting of salts and polymers.

11. The method according to claim 10, wherein the polymer is an anionic or a cationic polymer.

12. The method according to any of the preceding claims, wherein the poly aluminium chloride is of the formula Alₙ(OH)ₘCl(₃ₙ₋ₘ).

13. The method according to claim 12, wherein the poly aluminium chloride is of the formula Al₂(OH)₅Cl and/or Al(OH)_{1,2}Cl_{1,8}.

14. The method according to any of the preceding claims, wherein the separation in step a) or b) is performed by centrifugation or filtration.

15. The method according to any of the preceding claims, wherein pH is adjusted to a pH within the range of pH 2 to pH 11 after addition of the poly aluminium chloride.

16. The method according to any of the preceding claims, wherein there is less than 10 ng host cell DNA per gram fermentation broth after step b).

17. The method according to any of the preceding claims, wherein there is less than 10 ng host cell DNA per gram fermentation broth after step b) and/or c), when subjected to PCR reaction and gel-electrophoresis after step b) and/or c).

18. The method according to any of the preceding claims, wherein there is below detection limit of host cell DNA in the fermentation broth when subjected to PCR reaction and gel-electrophoresis after step b) and/or c).

## Patentansprüche

1. Verfahren zum Entfernen von restlicher Wirtszell-DNA aus einer Fermentationsbrühe, die ein Protein von Interesse und einen Mikroorganismus umfasst, der das Protein von Interesse herstellt, wobei das Verfahren umfasst:
a) Zugeben eines Polyaluminiumchlorids zur Fermentationsbrühe, und
b) Trennen des ausgeflockten Mikroorganismus von der Fermentationsbrühe.

2. Verfahren zum Entfernen von restlicher Wirtszell-DNA aus einer Fermentationsbrühe, die ein Protein von Interesse und einen Mikroorganismus umfasst, der das Protein von Interesse herstellt, wobei das Verfahren umfasst:
a) Trennen des Mikroorganismus von der Fermentationsbrühe; und
b) Zugeben eines Polyaluminiumchlorids zur Fermentationsbrühe.

3. Verfahren zum Entfernen von restlicher Wirtszell-DNA aus einer Fermentationsbrühe und anschließenden nachgeordneten Verfahrensflüssigkeiten, die ein Protein von Interesse und einen Mikroorganismus umfassen, der das Protein von Interesse herstellt, wobei das Verfahren umfasst:
a) Zugeben eines Polyaluminiumchlorids zur Fermentationsbrühe und zu den anschließenden nachgeordneten Verfahrensflüssigkeiten;
b) Trennen des ausgeflockten Mikroorganismus von der Fermentationsbrühe; und
c) Aufbereiten der anschließenden nachgeordneten Verfahrensflüssigkeit nach Zugeben des Polyaluminiumchlorids.

4. Verfahren nach einem beliebigen der voranstehenden Ansprüche, wobei das Protein ein Enzym ist.

5. Verfahren nach einem beliebigen der voranstehenden Ansprüche, wobei der Mikroorganismus ein Bakterium oder ein Pilz ist.

6. Verfahren nach einem beliebigen der voranstehenden Ansprüche, wobei das Enzym eine Hydrolase ist.

7. Verfahren nach einem beliebigen der voranstehenden Ansprüche, wobei die Fermentationsbrühe bis zu 2000% (Gew./Gew.) mit Wasser verdünnt sein kann.

8. Verfahren nach einem beliebigen der voranstehenden Ansprüche, wobei das Polyaluminiumchlorid in einer Konzentration von 0,1-10% (Gew./Gew.) berechnet pro kg Fermentationsbrühe zugegeben wird.

9. Verfahren nach einem beliebigen der voranstehenden Ansprüche, wobei ein oder mehrere Flockungsmittel zusätzlich zum Polyaluminiumchlorid zugegeben werden.

10. Verfahren nach Anspruch 9, wobei die Flockungsmittel aus der Gruppe bestehend aus Salzen und Polymeren ausgewählt sind.

11. Verfahren nach Anspruch 10, wobei das Polymer ein anionisches oder ein kationisches Polymer ist.

12. Verfahren nach einem beliebigen der voranstehenden Ansprüche, wobei das Polyaluminiumchlorid von der Formel Alₙ(OH)ₘC(₃ₙ₋ₘ) ist.

13. Verfahren nach Anspruch 12, wobei das Polyaluminiumchlorid von der Formel Al₂(OH)₅Cl und/oder Al(OH)_{1,2}Cl_{1,8} ist.

14. Verfahren nach einem beliebigen der voranstehenden Ansprüche, wobei die Trennung in Schritt a) oder b) durch Zentrifugation oder Filtration ausgeführt wird.

15. Verfahren nach einem beliebigen der voranstehenden Ansprüche, wobei der pH auf einen pH im Bereich von pH 2 bis pH 11 nach Zugabe des Polyaluminiumchlorids eingestellt wird.

16. Verfahren nach einem beliebigen der voranstehenden Ansprüche, wobei weniger als 10 ng Wirtszelle-DNA pro Gramm Fermentationsbrühe nach Schritt b) vorliegen.

17. Verfahren nach einem beliebigen der voranstehenden Ansprüche, wobei weniger als 10 ng Wirtszelle-DNA pro Gramm Fermentationsbrühe nach Schritt b) und/oder c) vorliegen, wenn sie einer PCR-Reaktion und Gelelektrophorese nach Schritt b) und/oder c) unterzogen wird.

18. Verfahren nach einem beliebigen der voranstehenden Ansprüche, wobei Wirtszell-DNA in der Fermentationsbrühe unterhalb der Nachweisgrenze vorliegt, wenn sie einer PCR-Reaktion und Gelelektrophorese nach Schritt b) und/oder c) unterzogen wird.

## Revendications

1. Méthode pour éliminer l'ADN résiduel de cellule hôte d'un bouillon de fermentation comprenant une protéine d'intérêt et un microorganisme produisant la protéine d'intérêt, ladite méthode comprenant :
a) l'addition d'un polychlorure d'aluminium au bouillon de fermentation, et
b) la séparation du microorganisme floculé d'avec le bouillon de fermentation.

2. Méthode pour éliminer l'ADN résiduel de cellule hôte d'un bouillon de fermentation comprenant une protéine d'intérêt et un microorganisme produisant la protéine d'intérêt, ladite méthode comprenant :
a) la séparation du microorganisme d'avec le bouillon de fermentation ; et
b) l'addition d'un polychlorure d'aluminium au bouillon de fermentation.

3. Méthode pour éliminer l'ADN résiduel de cellule hôte d'un bouillon de fermentation et des liquides de procédé en aval subséquents comprenant une protéine d'intérêt et un microorganisme produisant la protéine d'intérêt, ladite méthode comprenant :
a) l'addition d'un polychlorure d'aluminium au bouillon de fermentation et aux liquides de procédé en aval subséquents ;
b) la séparation du microorganisme floculé d'avec le bouillon de fermentation ; et
c) le raffinage du liquide de procédé en aval subséquent après addition du polychlorure d'aluminium.

4. Méthode selon l'une quelconque des revendications précédentes, dans laquelle la protéine est une enzyme.

5. Méthode selon l'une quelconque des revendications précédentes, dans laquelle le microorganisme est une bactérie ou un champignon.

6. Méthode selon l'une quelconque des revendications précédentes, dans laquelle l'enzyme est une hydrolase.

7. Méthode selon l'une quelconque des revendications précédentes, dans laquelle le bouillon de fermentation peut être dilué jusqu'à 2 000 % (p/p) avec de l'eau.

8. Méthode selon l'une quelconque des revendications précédentes, dans laquelle le polychlorure d'aluminium est ajouté à une concentration de 0,1 à 10 % (p/p), calculée par kg de bouillon de fermentation.

9. Méthode selon l'une quelconque des revendications précédentes, dans laquelle un ou plusieurs agents de floculation sont ajoutés en plus du polychlorure d'aluminium.

10. Méthode selon la revendication 9, dans laquelle les agents de floculation sont choisis dans le groupe constitué par les sels et les polymères.

11. Méthode selon la revendication 10, dans laquelle le polymère est un polymère anionique ou cationique.

12. Méthode selon l'une quelconque des revendications précédentes, dans laquelle le polychlorure d'aluminium est de formule Alₙ(OH)ₘCl(₃ₙ₋ₘ).

13. Méthode selon la revendication 12, dans laquelle le polychlorure d'aluminium est de formule Al₂(OH)₅Cl et/ou Al(OH)_{1,2}Cl_{1,8}.

14. Méthode selon l'une quelconque des revendications précédentes, dans laquelle la séparation dans l'étape a) ou b) est effectuée par centrifugation ou filtration.

15. Méthode selon l'une quelconque des revendications précédentes, dans laquelle le pH est ajusté à un pH situé dans la plage allant de pH 2 à pH 11 après l'addition du polychlorure d'aluminium.

16. Méthode selon l'une quelconque des revendications précédentes, dans laquelle il y a moins de 10 ng d'ADN de cellule hôte par gramme de bouillon de fermentation après l'étape b).

17. Méthode selon l'une quelconque des revendications précédentes, dans laquelle il y a moins de 10 ng d'ADN de cellule hôte par gramme de bouillon de fermentation après les étapes b) et/ou c), lors d'une soumission à une réaction PCR et à une électrophorèse sur gel après les étapes b) et/ou c).

18. Méthode selon l'une quelconque des revendications précédentes, dans laquelle l'ADN de cellule hôte est inférieur à la limite de détection dans le bouillon de fermentation lors d'une soumission à une réaction PCR et à une électrophorèse sur gel après les étapes b) et/ou c).
